# EUROPEAN PATENT APPLICATION

(11) **EP 1 967 194 A1**
(43) Date of publication of application: **10.09.2008**
(21) Application number: 06841724.5
(22) Date of filing: 01.12.2006
(51) Int. Cl.: A61K 33/14, A61K 9/08, A61P 1/10

(54) **RECTAL USE OF A SODIUM CHLORIDE SOLUTION AS AN INTESTINAL CLEANSER**

(30) Priority: 02.12.2005 ES 200502995
(71) Applicant: Laboratorios Casen-Fleet, S.L., 50180 Utebo, Zaragoza (ES)
(72) Inventor: TABUENCA NAVARRO, Daniel, E-50180 Utebo (ES)
(74) Representative: Ungria Lopez, Javier
(86) International application number: PCT/ES2006/000672
(87) International publication number: WO 2007/063159

(57) **Abstract**

Rectal use of a sodium chloride solution between 0.5% and 1.5% for rectal use, usable as an intestinal cleaner by simple mechanical action, used as an intestinal evacuator or cleaner in all those cases where intestinal evacuation is required, but without having any therapeutic effect, thus, avoiding side effects.

## Description

### OBJECT OF THE INVENTION

As expressed in the title of the preset patent specification, the following invention refers to rectal use of a sodium chloride solution as a intestinal cleaner, of the type of an aqueous sodium chloride solution, so that the sodium chloride solution for rectal use, used as an intestinal cleaner, has the essential purpose of being able to be applied in all those cases where intestinal evacuation is necessary.

Hence, the rectal application of sodium chloride solution between 0.5 and 1.5% as an intestinal cleaner is recommendable in any state of the patient given that the sodium chloride solution between 0.5 and 1.5% acts as an intestinal cleaner by mechanical action, lacking active principles, thus, there is no therapeutic effect and thus there are no side effects.

### FIELD OF USE

The sodium chloride solution between 0.5 and 1.5% is especially applicable as a rectal intestinal cleaner upon being presented in the corresponding container appropriate for this effect.

### BACKGROUND OF THE INVENTION

As it is known, on different occasions it becomes necessary to carry out intestinal evacuation of a patent and there are different enemas on the market for rectal use. They are comprised of different active principles.

Hence, intestinal evacuation is necessary in cases such as before and after surgery, before and after delivery; before having a proctoscopy, colonscopy and signoid-scopy (exploration of the intestine by endoscopic techniques using a probe); before X-rays, fecal impactation, etc.

On the other hand, it should be taken into account that an enema with active principles is a medicine, whose active principles can have side effects.

In this way, in principle, it is recommended not to use the same in children younger than two and the dose recommended for healthy people, according to the doctor's prescription, varies in terms of age/weight, while in those cases where a patient may suffer from certain disorders, such as kidney failure, an enema with active principles should not be administered.

Besides, in those cases when a patient is urgently taken into the hospital with some symptom that affects the intestine and above all, it is an elderly person, although in principle administration of an enema with active principles may be recommendable, the same may end up being counterproductive.

As to the known "physiological serum" comprised of an aqueous 0.9% sodium chloride solution with the same isotonicity as body liquids, are present in different pharmaceutical forms but not for rectal use.

Hence "physiological serum" presented in a container is very useful in young children in order to dissolve nasal mucous and, likewise, it is very useful for cleaning eyes.

### DESCRIPTION OF THE INVENTION

The present specification presents a sodium chloride solution between 0.5% and 1.5% for rectal use, usable as an intestinal cleaner by simple mechanical action.

Hence, the sodium chloride solution between 0.5% and 1.5%, for rectal use, is used as an intestinal evacuator or cleaner, in all cases in which intestinal evacuation is necessary.

The sodium chloride solution between 0.5 and 1.5%, for rectal use, is used as an intestinal cleaner by simple mechanical action, having no therapeutic effect, thus it lacks side effects. It may be used in any situation where it is recommended to evacuate the intestine.

In a preferred embodiment, the solution has 0.9% by weight of sodium chloride.

In this way, the sodium chloride solution between 0.5 and 1.5% of rectal use, will be able to be used as an enema in very young children, even breast-feeders.

Likewise, the sodium chloride solution between 0.5 and 1.5% for rectal use, will be able to be used as an enema in elderly people, although they have some rectal alteration, as well as even if there are problems of dehydration, since, the only action on the body is to clean the intestine, which represents a very important advantage.

In this way, the sodium chloride solution between 0.5% and 1.5%, for rectal use, will also be able to be used as an enema in elderly people who have some other clinical problem which makes it advisable not to apply an enema with active principles.

In this way, before any need to carry out urgent intestinal evacuation, the invention may be used without any problem, since, evacuation is produced by simple mechanical action and there are no side effects.

The sodium chloride solution between 0.5% and 1.5% as an enema will be presented in the corresponding flexible container of different capacities, sealed and provided with the respective non-return valve and rectal cannula. It is recommended in all those cases when it is necessary to evacuate the intestine.

In this way, the sodium chloride solution between 0.5 and 1.5% used in the rectum, as an intestinal cleaner, will be useful even when a patient has:
Kidney failure
Heart failure
Intestinal occlusion
Congenital or acquired megacolon
Unspecific constipation, etc.

Given that it acts by simple mechanical action and there are no side effects.

A preservative may or may not be added to this sodium chloride solution of 0.5% and 1.5%.

## Claims

1. Rectal use of a sodium chloride solution, being of the type of sodium chloride solution used as physiological serum, **characterized in that** sodium chloride solution between 0.5% and 1.5% is administered through the rectum.

2. Rectal use of a sodium chloride solution, according to claim 1, **characterized in that** the sodium chloride solution between 0.5% and 1.5% is used as an intestinal cleaner.

3. Rectal use of a sodium chloride solution, according to claims 1 and 2, **characterized in that** the sodium chloride solution between 0.5 % and 1.5 % is used as an intestinal cleaner by simple mechanical action.

4. Rectal use of a sodium chloride solution, according to the preceding claims, **characterized** because the sodium chloride solution has 0.9% sodium chloride by weight.

5. Rectal use of a sodium chloride solution, according to claim 1, **characterized** because the sodium chloride solution between 0.5 and 1.5% is used as an enema in very young children and even breast-feeders.

6. Rectal use of a sodium chloride solution, according to claim 1, **characterized** because the sodium chloride solution between 0.5% and 1.5% is used as an enema in elderly people.
